(19) Europäisches Patentamt
European Patent Office
Office européen des brevets

(11) **EP 3 036 518 B1**

(12) **EUROPÄISCHE PATENTSCHRIFT**

(45) Veröffentlichungstag und Bekanntmachung des Hinweises auf die Patenterteilung:
**10.10.2018 Patentblatt 2018/41**

(21) Anmeldenummer: **14750496.3**

(22) Anmeldetag: **13.08.2014**

(51) Int Cl.:
*F02D 41/14* (2006.01)          *G01N 21/3504* (2014.01)
*G01N 33/00* (2006.01)          *G01M 3/00* (2006.01)
*G01M 3/20* (2006.01)

(86) Internationale Anmeldenummer:
**PCT/EP2014/067309**

(87) Internationale Veröffentlichungsnummer:
**WO 2015/024831 (26.02.2015 Gazette 2015/08)**

(54) **PICO-PRÜFLECK**

PICO TEST LEAK

PICO-FUITE ÉTALON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.08.2013 DE 102013216450**

(43) Veröffentlichungstag der Anmeldung:
**29.06.2016 Patentblatt 2016/26**

(73) Patentinhaber: **Inficon GmbH**
**50968 Köln (DE)**

(72) Erfinder: **WIDT, Rudi**
**50969 Köln (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
**EP-A1- 0 110 004          EP-A1- 1 637 861**
**EP-A2- 0 242 684          EP-A2- 0 352 371**
**DE-A1- 4 038 266          DE-A1- 19 906 941**
**DE-A1-102009 012 213      US-A- 5 663 487**

**Beschreibung**

Die Erfindung betrifft eine Prüfleckvorrichtung zum Testen und Kalibrieren eines Gaslecksuchgeräts.

Prüflecks weisen ein mit einem Prüfgas gefülltes Gehäuse auf und sind mit einem Leck versehen, das eine vorgegebene, bekannte Leckrate aufweist, durch die das Prüfgas nach außen dringt. Die Funktionsfähigkeit oder Genauigkeit eines Gaslecksuchgeräts kann getestet werden, indem das aus dem Prüfleck austretende Gas gemessen wird.

In EP 0 742 894 B1 ist ein mit einer Kapillare versehenes Prüfleck beschrieben, welches eine vergleichsweise geringe Leckrate von $10^{-7}$ mbarl/s oder mehr erzielt. Derartige Kapillare können nicht beliebig klein ausgebildet werden, weil sie sonst durch Luftfeuchtigkeit verstopfen und unbrauchbar werden. Mit den bekannten Prüflecks ist es bislang nicht möglich gewesen, Leckraten von $10^{-12}$ mbarl/s oder weniger zu erzeugen. Derartige Leckraten werden benötigt, um Dichtheitsprüfgeräte für sehr kleine Lecks zu testen.

EP 0 352 371 A2 beschreibt einen Heliumleckdetektor mit einer nur für Helium permeablen Kapillare, die über eine Ionenpumpe mit dem Detektor verbunden ist.

EP 1 637 861 A1 beschreibt ein Referenzgasleck mit einer Röhre, die einen Stopfen aufweist, der mit einer Bohrung versehen ist.

DE 4 038 266 A1 beschreibt eine Vorrichtung zur Prüfung der Gasdichtigkeit von Bauteilen, mit einer Vakuumtestkammer und einem Gasmessgerät.

EP 0 110 004 A1 beschreibt ein der Kontrolle oder dem Abgleich von Lecksuchgeräten dienendes Testleck mit einer Engstelle, die die Leckrate des Testlecks bestimmt.

US 5,663,487 beschreibt eine Kapillarröhre zur Bestimmung der Leckrate eines Testlecks.

EP 0 242 684 A2, DE 10 2009 012 213 A1 und DE 19906941 A1 beschreiben jeweils Testlecks für Lecksuchgeräte.

Der Erfindung liegt die Aufgabe zugrunde, ein Prüfleck mit einer geringen Leckrate im Bereich von $10^{-12}$ mbarl/s bereitzustellen.

Die erfindungsgemäße Prüfleckvorrichtung ist definiert durch die Merkmale von Patentanspruch 1.

Die erfindungsgemäße Prüfleckvorrichtung weist einen mit einem Gas, das zumindest zu 10 Vol. % aus atmosphärischer Luft besteht, gefüllten Behälter auf. Der übrige Anteil des Gases kann zum Beispiel Stickstoff sein. Die Behälterwand wird von mindestens einer Kapillare durchdrungen. Die Kapillare sollte eine Leckrate im Bereich von etwa $10^{-5}$ bis $10^{-7}$ mbarl/s und vorzugsweise etwa $10^{-6}$ mbarl/s aufweisen. Atmosphärische Luft führt nicht zu einem Verstopfen der Kapillare und weist typischerweise einen Anteil von Helium und einen Anteil von Argon auf. Helium und Argon sind typische Prüfgase in der Gaslecksuche. Der Anteil von Helium an der in dem Behälter enthaltenen Luft sollte im Bereich zwischen drei und sieben ppm und vorzugsweise etwa 3,5 bis 5,5 ppm betragen. Ein besonders vorteilhafter Heliumanteil ist etwa 5 ppm. Mit "etwa" ist in der vorliegenden Beschreibung jeweils eine Abweichung von $\pm 10$ % gemeint. Der Argonanteil an der in dem Behälter enthaltenen Luft sollte im Bereich von 0,1 bis 2 % und vorzugsweise etwa 0,8 bis 1,2 % des Volumens betragen. Ein besonders vorteilhafter Argonanteil ist etwa 1 %.

Die Füllung des Prüfleckbehälters mit Atmosphärenluft erübrigt zudem einen separaten Befüllstutzen an dem Prüfleck, der die äußeren Abmessungen erhöhen und den Einsatz in kleinen Prüfkammern erschweren würde.

Besonders vorteilhaft ist es, wenn es sich bei dem Behälter um einen Zylinder mit einem stirnseitigen Deckel beziehungsweise einer Stirnkappe handelt. Die Stirnkappe ist von der Kapillare durchdrungen. Der Zylinder sollte eine Länge von 5 cm und einen Durchmesser von etwa 1 cm nicht überschreiten. Die Kapillare kann innerhalb des Zylinders entlang dessen Mittellängsachse geführt sein. Besonders vorteilhaft ist es, wenn der Behälter aus Glas und die Stirnkappe aus einem Metall bestehen. Ein solcher Behälter kann auch in besonders kleinen Prüfkammern eingesetzt werden und auf einfache Weise befüllt werden. Das Befüllen erfolgt einfach dadurch, dass die Stirnkappe abgenommen wird und das Gas mit der atmosphärischen Luft in den Behälter einströmt. Der zylindrische Prüfleckbehälter weist eine geringe Oberfläche auf und ist aufgrund fehlender Spalte oder Vertiefungen einfach in einer Prüfkammer zu evakuieren.

Das Gas in dem Behälter sollte eine relative Luftfeuchtigkeit von weniger als 50 % und vorzugsweise von weniger als 40 % aufweisen, um ein Verstopfen der Kapillare aufgrund von Luftfeuchtigkeit zu vermeiden.

Im Folgenden wird anhand der Figur ein Ausführungsbeispiel näher erläutert. Die Figur zeigt einen Längsschnitt durch die Prüfleckvorrichtung.

Die Prüfleckvorrichtung 10 besteht aus einem zylindrischen Behälter 12 aus Glas mit einem geschlossenen Boden 14 an einer Stirnseite und einem offenen Ende an der gegenüberliegenden Stirnseite. Das offene Ende ist mit einer Stirnkappe 16 aus Metall dichtend verschlossen. Durch die Metallkappe 16 ist eine Kapillare 18 hindurch entlang der Mittellängsachse des Zylinders 12 geführt.

Der Behälter 12 ist mit atmosphärischer Luft 20 gefüllt, die einen Heliumanteil von 5 ppm und einen Argonanteil von 1 Vol. % aufweist. Die Kapillare 18 weist eine Leckrate von $10^{-6}$ mbarl/s auf. Die Feuchtigkeit der Luft beträgt 40 %. Für die Prüfleckvorrichtung 10 ergibt sich daraus ein effektiver Gasfluss von

$$1 \cdot 10^{-6} \text{ mbarl/s} \cdot 5 \text{ ppm} = 5 \cdot 10^{-12} \text{ mbarl/s für Helium}$$

und

$$1 \cdot 10^{-6} \text{ mbarl/s} \cdot 1 \% = 1 \cdot 10^{-8} \text{ mbarl/s für Argon.}$$

**Patentansprüche**

1. Prüfleckvorrichtung (10) zum Testen oder Kalibrieren eines Lecksuchgeräts, mit einem gasgefüllten Behälter (12) und einer die Behälterwand durchdringenden Kapillare (18), die eine vorgegebene, bekannte Leckrate von maximal $1 \cdot 10^{-6}$ mbarl/s aufweist, durch die das Gas nach außen dringt,
**dadurch gekennzeichnet,**
**dass** das Gas zumindest zu einem Volumenanteil von 10 % aus atmosphärischer Luft (20) mit einer relativen Feuchtigkeit von weniger als 50 % besteht und
**dass** der Behälter (12) ein zylindrischer Behälter (12) mit einer abnehmbaren Stirnkappe (16) ist, durch die die Kapillare (18) geführt ist.

2. Prüfleckvorrichtung (10) nach Anspruch 1, **dadurch gekennzeichnet, dass** die atmosphärische Luft (20) eine relative Feuchtigkeit von weniger als 40 % aufweist.

3. Prüfleckvorrichtung (10) nach einem der vorhergehenden Ansprüche, **dadurch gekennzeichnet, dass** die Luft (20) einen Heliumvolumenanteil im Bereich zwischen 3 ppm und 7 ppm aufweist.

4. Prüfleckvorrichtung (10) nach dem vorhergehenden Anspruch, **dadurch gekennzeichnet, dass** die Luft (20) einen Heliumvolumenanteil im Bereich zwischen etwa 4,5 ppm und 5,5 ppm aufweist.

5. Prüfleckvorrichtung (10) nach einem der vorhergehenden Ansprüche, **dadurch gekennzeichnet, dass** die Luft (20) einen Argonvolumenanteil von etwa 0,5 % bis 2 % aufweist.

6. Prüfleckvorrichtung (10) nach dem vorhergehenden Anspruch, **dadurch gekennzeichnet, dass** die Luft einen Argonvolumenanteil von etwa 0,8 % bis 1,2 % aufweist.

7. Prüfleckvorrichtung (10) nach einem der vorhergehenden Ansprüche, **dadurch gekennzeichnet, dass** der Zylinder (12) aus Glas besteht.

8. Prüfleckvorrichtung (10) nach einem der vorhergehenden Ansprüche, **dadurch gekennzeichnet, dass** die Kappe (16) aus einem Metall besteht.

9. Prüfleckvorrichtung (10) nach einem der vorhergehenden Ansprüche, **dadurch gekennzeichnet, dass** der Behälter (12) eine Länge von maximal 5 cm und einen Durchmesser von maximal 1 cm aufweist.

10. Prüfleckvorrichtung (10) nach dem vorhergehenden Anspruch, **dadurch gekennzeichnet, dass** der Behälter (12) eine Länge von etwa 4 cm und einen Durchmesser von etwa 0,8 cm aufweist.

**Claims**

1. A test leak device (10) for testing or calibrating a leak detecting apparatus, the device having a gas-filled container (12) and a capillary (18) penetrating a container wall, which has a predetermined known leakage rate of at most $10^{-6}$ mbar·l/s, the gas escaping outward through the capillary,
**characterized in**
**that** the proportion of atmospheric air (20) in the gas is at least 10% with a relative humidity of less than 50%, and
**that** the container (12) is a cylindrical container (12) with a removable end cap (16) through which the capillary (18) is guided.

**2.** The test leak device (10) of claim 1, **characterized in that** the atmospheric air (20) has a relative humidity of less than 40%.

**3.** The test leak device (10) of one of the preceding claims, **characterized in that** the proportion of helium in the air (20) is in a range from 3 ppm to 7 ppm.

**4.** The test leak device (10) of the preceding claim, **characterized in that** the proportion of helium in the air (20) is in a range from about 4.5 ppm to 5.5 ppm.

**5.** The test leak device (10) of one of the preceding claims, **characterized in that** the proportion of argon in the air (20) is about 0.5% to 2%.

**6.** The test leak device (10) of the preceding claim, **characterized in that** the proportion of argon in the air is about 0.8% to 1.2%.

**7.** The test leak device (10) of one of the preceding claims, **characterized in that** the cylinder (12) is made of glass.

**8.** The test leak device (10) of one of the preceding claims, **characterized in that** the cap (16) is made of a metal.

**9.** The test leak device (10) of one of the preceding claims, **characterized in that** the container (12) has a length of 5 cm at most and a diameter of 1 cm at most.

**10.** The test leak device (10) of the preceding claim, **characterized in that** the container (12) has a length of about 4 cm and a diameter of about 0.8 cm.


**Revendications**

**1.** Testeur de fuite (10), destiné à tester ou étalonner un détecteur de fuite, comprenant un récipient (12) rempli de gaz et un capillaire (18) qui pénètre à travers la paroi du récipient qui a une vitesse de fuite connue prédéterminée de $1 \cdot 10^{-6}$ barl/s maximum et par lequel le gaz s'échappe vers l'extérieur,
**caractérisé en ce que**
le gaz est constitué, pour au moins 10 % en volume, d'air atmosphérique (20) avec une humidité relative inférieure à 50 %, et
le récipient (12) est un récipient cylindrique (12) muni d'un capuchon d'extrémité amovible (16) à travers lequel le capillaire (18) est guidé.

**2.** Testeur de fuite (10) selon la revendication 1, **caractérisé en ce que** l'air atmosphérique (20) a une humidité relative inférieure à 40 %.

**3.** Testeur de fuite (10) selon l'une quelconque des revendications précédentes, **caractérisé en ce que** l'air (20) comporte une proportion en volume d'hélium dans la gamme comprise entre 3 ppm et 7 ppm.

**4.** Testeur de fuite (10) selon la revendication précédente, **caractérisé en ce que** l'air (20) comporte une proportion en volume d'hélium dans la gamme comprise entre environ 4,5 ppm et 5,5 ppm.

**5.** Testeur de fuite (10) selon l'une des revendications précédentes, **caractérisé en ce que** l'air (20) comporte une proportion en volume d'argon d'environ 0,5 % à 2 %.

**6.** Testeur de fuite (10) selon la revendication précédente, **caractérisé en ce que** l'air comporte une proportion en volume d'argon d'environ 0,8 % à 1,2 %.

**7.** Testeur de fuite (10) selon l'une des revendications précédentes, **caractérisé en ce que** le cylindre (12) est en verre.

**8.** Testeur de fuite (10) selon l'une des revendications précédentes, **caractérisé en ce que** le capuchon (16) est constitué d'un métal.

**9.** Testeur de fuite (10) selon l'une des revendications précédentes, **caractérisé en ce que** le récipient (12) a une longueur maximale de 5 cm et un diamètre maximum de 1 cm.

10. Testeur de fuite (10) selon la revendication précédente, **caractérisé en ce que** le récipient (12) a une longueur d'environ 4 cm et un diamètre d'environ 0,8 cm.

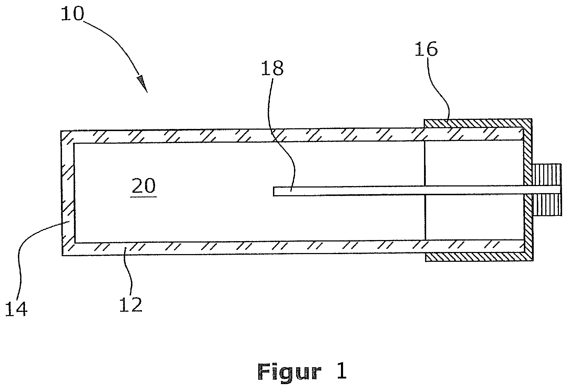

**Figur 1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0742894 B1
- EP 0352371 A2
- EP 1637861 A1
- DE 4038266 A1
- EP 0110004 A1

- US 5663487 A
- EP 0242684 A2
- DE 102009012213 A1
- DE 19906941 A1